Europäisches Patentamt

European Patent Office

Office européen des brevets

(19)

(11) Publication number:

**0 325 471**
**A1**

## EUROPEAN PATENT APPLICATION

(12)

(21) Application number: **89300532.2**

(22) Date of filing: **20.01.89**

(51) Int. Cl.⁴: **A 61 K 37/36**
**A 61 K 45/02**
**//(A61K37/36,37:02),**
**(A61K45/02,37:36)**

(30) Priority: **22.01.88 US 146901**

(43) Date of publication of application:
**26.07.89 Bulletin 89/30**

(84) Designated Contracting States:
**AT BE CH DE ES FR GB GR IT LI LU NL SE**

(71) Applicant: **COLLAGEN CORPORATION**
**2500 Faber Place**
**Palo Alto, California 94303-3334 (US)**

(72) Inventor: **Ellingsworth, Larry Ross**
**3716 Springbook Avenue**
**San Jose, California 95148 (US)**

**Ruscetti Francis W.**
**10201 Grosvenor Place No 1510**
**Rockville, Maryland 20852 (US)**

(74) Representative: **Harrison, David Christopher et al**
**MEWBURN ELLIS & CO 2/3 Cursitor Street**
**London EC4A 1BQ (GB)**

Claims for the following Contracting States: ES + GR.
A request for addition of a second applicant, "National Institute of Health", has been filed pursuant to Rule 88 EPC. A decision on the request has not yet been taken.

(54) **Method for suppressing the growth of normal and cancer cells.**

(57) Transforming Growth Factor-b (TGF-b) enhances the growth-inhibiting activity of tumor necrosis factor (TNF) or interferon-a (IFN-a) on immune cells and hematopoietic progenitor cells, as well as cancer cells. Thus, combinations of TGF-b with TNF or IFN-a are effective for suppressing immune cell and hematopoietic progenitor cell growth, and are thus useful for treating cancer (e.g., lymphomas, leukemias, myelomas, adenocarcinomas, etc.), autoimmune and chronic inflammatory diseases (e.g., rheumatoid arthritis, osteoarthritis, myasthenia gravis, lupus erythematosus, uveitis, etc.) hyperproliferative diseases (e.g., psoriasis vulgaris), and organ transplant rejection, and for protecting immune cells and hematopoietic progenitor cells from the lethal effects of cancer chemotherapy and radiotherapy.

EP 0 325 471 A1

## Description

## METHOD FOR SUPPRESSING THE GROWTH OF NORMAL AND CANCER CELLS

The technical field of the invention relates to regulation of the growth and development of immune and hematopoietic cells, and to the suppression of malignant cell growth. More specifically, it relates to combinations of peptide factors comprising transforming growth factor-beta (TGF-b) and certain cytokines, which are more effective at suppressing normal and malignant cell growth than currently known treatments. Such combinations are functionally and therapeutically more effective than individual cytokines, and exhibit fewer side effects (e.g., lower toxicity). The invention also relates to a method for suppressing hematopoietic progenitor cell growth comprising administering a therapeutically effective amount of TGF-b with tumor necrosis factor (TNF) or alpha-interferon (IFN-a). A method for suppressing immune cell function comprising administering a therapeutically effective amount of TGF-b with TNF or IFN-a is also an aspect of this invention. This invention also relates to a method for suppressing malignant cell growth comprising administering a therapeutically effective amount of TGF-b and TNF. The compositions of this invention are useful in various therapeutic applications, including but not limited to, protection of bone marrow stem cells from lethal doses of irradiation and cell cycle-dependent cytotoxic drugs, treatment of autoimmune and hyperproliferative diseases, prevention of transplant rejection, and treatment of various types of cancer.

The body's main defenses against disease and cancer are cells of the immune and hematopoietic systems. The growth and function of immune and hematopoietic cells are regulated by complex interactions between many different cells. These interactions are controlled by small molecular weight protein factors known as cytokines.

Cytokines generally function to stimulate or suppress cell growth. Mitogenic or differentiation factors are cytokines which stimulate the growth of cells. Cytokines that inhibit cell growth are known as suppressor factors. Autocrine factors are cytokines that affect the cells expressing them. Cytokines that are produced by one cell type and act upon a different cell type are known as paracrine factors.

Suppressor factors which act to inhibit the growth and function of the immune and hematopoietic systems have many therapeutic applications, including modulation of chronic inflammatory and hyperproliferative diseases, treatment of various autoimmune diseases, and prevention of homograft and transplant rejection. In addition, suppressor factors are also useful in the treatment of cancer where cell growth is uncontrolled. The latter application includes, but is not limited to, lymphomas, myelomas, leukemias, melanomas, and adenocarcinomas. Examples of growth inhibitors are TGF-b, TNF, and IFN.

There are various forms of TNF. TNF-a, also known as cachectin (Beutler et al., (1985) Nature, 316:553), is a monocyte/macrophage cytokine which is produced in large quantities in response to endotoxin or other stimuli that simulates host invasion. The cachectin activity of TNF-a appears to arise from specific inhibition of lippoprotein lipase expression in adipocytes (Torti et al., (1985) Science, 229:867). In addition to its cytotoxic effect on tumor cell lines, TNF-a exhibits cytokine-like activity on many normal cell types. It induces expression of type 1 histocompatability antigens and other surface markers on normal endothelial cells, enhances the growth of fibroblasts, stimulates the production of collagenase and prostaglandin $E_2$, and stimulates release of interleukin-1 (IL-1) by endothelial cells.

Interferons are a large group of proteins historically classified based on their cell lineage. Thus IFN-a derives from leukocytes, IFN-b derives from fibroblasts, and IFN-q derives from antigen-stimulated or mitogen-stimulated lymphocytes. Within these categories are subgroups, e.g., IFN-$b_1$ and IFN-$b_2$. Interferons were first characterized by their ability to inhibit or "interfere" with intracellular viral replication, and are now known to enhance the cytotoxicity of cytotoxic T-cells and macrophages.

Various forms of TGF-b have also been described. TGF-b has been shown to be very similar to a polypeptide known as growth inhibitor (GI), purified from BSC-1 monkey kidney cell-conditioned medium, and may be identical (Tucker, R.F. et al., (1984) Science 226:705). TGF-b and GI have both been shown to inhibit the growth of a variety of tumor cell lines (Assoian, R.K. et al., Cancer Cells 3/ Growth Factors and Transformation, Cold Spring Harbor Laboratory, June 1985, pages 59-64 and Moses, H.L. et al., Cancer Cells 3/ Growth Factors and Transformation, ibid, pages 65-71).

TGF-b derived from bovine kidney, human placenta, and human platelets is described in International Patent Application PCT/US83/01460, published 29 March 1984 under No. WO84/01106; and EPA 84450016.5, published 19 December 1984 under No. 0128849. These applications present data showing that TGF-b, when combined with EGF or TGF-a, promotes cell proliferation in a soft agar culture assay (inducing anchorage-independent growth of normal and rat kidney cell colonies in soft agar), and promotes cell proliferation and protein deposition in a rat soft tissue wound healing model.

The partial N-terminal amino acid sequence of human placenta-derived TGF-b is reported to be identical to bovine-derived cartilage-inducing factor-A (CIF-A). CIF-A is described in European Patent Application 85304848.6 (published 22 January 1986 under publication no. 0169016). The application also describes a homologous polypeptide designated as CIF-B. Both polypeptides have molecular weights of approximately 26,000 daltons (26Kd) by SDS-PAGE analysis and are dimers. The partial N-terminal sequence of CIF-B is different from that of CIF-A (eleven of the first 30 amino acids at the N-terminus are different). Both CIFs exhibit activity in the above mentioned TGF-b soft agar culture assay. Subsequently, CIF-A and CIF-B have become known in the art as TGF-b1 and TGF-b2, respectively.

In copending U.S. Patent Application Serial No. 836,672 filed 6 March 1986, both TGF-bs are described as possessing anti-inflammatory activity and as inhibiting mitogen-stimulated T cell proliferation and B cell activation. It is also reported that TGF-b is localized in centers of hematopoiesis and lymphopoiesis, and that TGF-b may therefore be useful for treating indications associated with malfunction or dysfunction of hematopoiesis or lymphopoiesis.

Copending U.S. Patent Application Serial No. 928,760 filed 7 November 1986 discloses that both TGF-bs exhibit oncostatic activity and may be used in treating any type of cellular neoplasm, including without limitation, carcinomas, myelomas, melanomas, and lymphomas.

We have now discovered particularly effective combinations of TGF-bs and cytokines that are useful in suppressing normal and malignant cell growth. TGF-b, when combined with TNF, is more effective at suppressing malignant cell growth than either TGF-b or TNF alone. Combinations of TFG-b with IFN-a or TNF are more effective at suppressing immune cell and hematopoietic progenitor cell growth than TGF-b, TNF, or IFN-a alone.

Combinations of TGF-b with specific cytokines, therefore, have therapeutic uses in the treatment of various diseases where inhibition of cell growth or immune function is necessary. These conditions include, but are not limited to, cancer (e.g., lymphomas, leukemias, myelomas, adenocarcinomas, etc.), autoimmune and chronic inflammatory diseases (e.g., rheumatoid arthritis, osteoarthritis, myasthenia gravis, lupus erythematosus, uveitis, etc.) hyperproliferative diseases (e.g., psoriasis vulgaris), and organ transplant rejection.

Summary of the Invention

One aspect of the invention is a therapeutically effective composition comprising TGF-b with TNF or IFN for inhibiting the proliferation of lymphoid, myeloid, or malignant cells in a mammal.

Another aspect of the invention is a method for suppressing the growth of hematopoietic progenitor cells and immune cells comprising administering to a mammal a therapeutically effective amount of TGF-b with TNF or IFN-a.

Yet another aspect of the invention is a method for inhibiting the proliferation of malignant cells comprising administering to a mammal a therapeutically effective amount of TGF-b with TNF.

Another aspect of the invention is a method of reducing the side effects of TNF or IFN-a treatment in a mammal, comprising reducing the amount of TNF or IFN-a administered, and coadministering a therapeutically effective amount of TGF-b.

Brief Description of the Drawings

Figure 1 is a graph of the results of TGF-b and TNF in the bone marrow soft agar assay described in Example 3(B).

Figure 2 is a graph of the results of TGF-b and TNF in the bone marrow proliferation assay described in Example 4(B).

Figure 3 is a graph of the results of TGF-b and IFN-a in the bone marrow proliferation assay described in Example 4(C).

Figure 4a is a graph of the results of TGF-b1 and IFN-a in the thymocyte proliferation assay described in Example 5(B).

Figure 4b is a graph of the results of TGF-b2 and IFN-a in the thymocyte proliferation assay described in Example 5(B).

Figure 5a is a graph of the results of TGF-b1 and IFN-b in the thymocyte proliferation assay described in Example 5(C).

Figure 5b is a graph of the results of TGF-b2 and IFN-b in the thymocyte proliferation assay described in Example 5(C).

Figure 6a is a graph of the results of TGF-b1 and IFN-q in the thymocyte proliferation assay described in Example 5(D).

Figure 6b is a graph of the results of TGF-b2 and IFN-q in the thymocyte proliferation assay described in Example 5(D).

Figure 7 is a graph of the results of TGF-b and TNF in the leukemic cell proliferation assay described in Example 6(B).

Detailed Description and Preferred Embodiments

The purification to homogeneity of TGF-b1 and TGF-b2 from demineralized bone and the characterization of the pure polypeptides are described in European Patent Application Publication No. 0169016, the disclosure of which is incorporated herein by reference.

TGF-b1 is apparently a homodimer having a molecular weight of approximately 26Kd as determined by SDS-PAGE. It has the following N-terminal amino acid sequence:

```
      1                  5                         10
Ala-Leu-Asp-Thr-Asn-Tyr-Cys-Phe-Ser-Ser-Thr-Glu-

              15        17             20
Lys-Asn-Cys-Cys-Val-Arg-Gln-Leu-Tyr-Ile-Asp-Phe-

25                            30
Arg-Lys-Asp-Leu-Gly-Trp-
```

TGF-b2 is also believed to be a homodimer of approximately 26Kd as measured by SDS-PAGE. Its N-terminal amino acid sequence is as follows:

```
1                       5                        10
Ala-Leu-Asp-Ala-Ala-Tyr-Cys-Phe-Arg-Asn-Val-Gln-

              15        17             20
Asp-Asn-(Cys-Cys-)-Leu-Arg-Pro-Leu-Tyr-Ile-Asp-

     25                      30
Phe-Lys-Arg-Asp-Leu-Gly-Trp-
```

Both TGF-bs are relatively insensitive (in terms of reduction in biological activity) to heat or trypsin treatment, but lose their activity on reduction with agents such as 2-mercaptoethanol or dithiothreitol.

The term "TGF-b" as used herein is intended to include the bovine polypeptides "TGF-b1" and "TGF-b2" described above, counterpart polypeptides derived from other mammalian species such as humans, pigs, sheep, and horses, and synthetic analogs (muteins) of either the bovine or other mammalian polypeptides. The analogs will typically be substantially similar in amino acid sequence (i.e., at least about 90% identity in sequence) to the particular native species. These TGF-bs may be derived from native sources or be prepared by recombinant DNA technology. Recombinant polypeptides may differ from the native polypeptide in manners other than in amino acid sequence, as is known in the art (e.g., lack of glycosylation).

Human TNF can be isolated from serum free tissue culture supernatants of the human myeloid leukemia cell line HL-60, such as that disclosed by Aggarwal, et al., J Biol Chem (1985) 260:2345. Recombinant forms of human and murine TNF are also available, as disclosed by Wang, et al. Science (1985) 228:149, and Pennica, et al., Proc Natl Acad Sci USA (1985) 82:6060.

The term "TNF" as used herein includes all molecules possessing TNF-like activity, regardless of the source of the material. Both TNF-a and TNF-b are included, along with synthetic analogs possessing a useful level of TNF-like activity. TNF-a is the preferred form in the practice of this invention.

The term "IFN" as used herein refers to all molecules exhibiting IFN-a-like activity. IFN-b and IFN-q, and other molecules that exhibit interferon-like activity but fail to be therapeutically effective with TGF-b, are not considered as within the scope of this invention. The purification and characterization of interferons are described in Methods of Enzymology, Volume 78, edited by Sidney Pestka, Academic Press, Inc., New York, NY, 1981.

The term "hematopoietic progenitor cells" as used herein refers to stem cells which give rise to the various cell types of the lymphoid and myeloid system.

The term "immune cells" as used herein refers to lymphocytes which are of the T-cell or B-cell phenotype.

The term "cancer cells" as used herein refers both benign and malignant neoplasms, and particularly to a tumor mass.

The term "substantially inhibits" as used herein refers to a complete or partial (at least 50%) arrest of normal or cancer cell growth.

The term "cell growth" as used herein includes the division and proliferation of cells.

The term "therapeutically effective" as used herein refers to the amount of TGF-b with TNF or IFN-a to substantially inhibit the growth of hematopoeitic progenitor cells, immune cells, and malignant cells. Such combinations are more effective at inhibiting cell growth than treatments of TGF-b, TNF, or IFN-a, alone. The precise quantity denoted by "therapeutically effective" amount will depend upon the particular cell type and the effect desired. As a general guideline, the concentration of TGF-b2 for preventing proliferation of C3H thymocytes in vitro can be as little as 0.00001 pM in the presence of 0.5 U/ml IFN-a, reducing T-cell proliferation by approximately 60%. By using 0.01 pM TGF-b2 with 50 U/ml IFN-a, one can suppress thymocyte proliferation by 95% or more. The therapeutically effective amount can also be defined in terms of the amount of TGF-b needed to reduce the dose amount of TNF or IFN-a to affect an inhibition of lymphoid, myeloid or malignant cell growth. By employing the assay methods disclosed herein, one of ordinary skill may determine the precise amounts of each component needed to achieve a therapeutically effective amount.

One aspect of the invention is a composition for suppressing the proliferation of hematopoietic progenitor cells, immune cells, and hyperproliferative cells in a mammal, which composition comprises a therapeutically effective amount of TGF-b with TNF or IFN-a. A preferred embodiment is a composition comprising a therapeutically effective amount of TGF-b with TNF-a; more preferably wherein TGF-b and TNF-a are administered in a ratio of one part TGF-b to 1-10,000 parts TNF-a, or one part TGF-b to 1-10,000 parts IFN-a.

Another aspect of the invention is a composition for inhibiting cancer cell growth in a mammal, which composition comprises a therapeutically effective amount of TGF-b and TNF. A preferred embodiment is a composition comprising a therapeutically effective amount of TGF-b and TNF, particularly TNF-a; more preferably wherein TGF-b and TNF-a are administered in a ratio of one part TGF-b to 1-10,000 parts TNF-a.

Another aspect of the invention is a method for suppressing the growth of hematopoietic progenitor cells, immune cells, and hyperproliferative cells, which method comprises administering to a mammal a therapeutically effective amount of TGF-b with TNF or IFN-a. A preferred embodiment comprises administering to a mammal a therapeutically effective amount of TGF-b and TNF, particularly TNF-a, more preferably wherein TGF-b and TNF-a are administered in a ratio of one part TGF-b to 1-10,000 parts TNF-a. Another preferred embodiment comprises administering to a mammal a therapeutically effective amount of TGF-b and IFN-a, more preferably wherein TGF-b and IFN-a are administered in a ratio of one part TGF-b to 1-10,000 parts IFN-a.

Another aspect of the invention is the method of inhibiting the growth of malignant cells, which method comprises administering to a mammal a therapeutically effective amount of TGF-b and TNF. A preferred embodiment is the method comprising administering to a mammal a therapeutically effective amount of TGF-b and TNF, particularly TNF-a, more preferably wherein TGF-b and TNF-a are administered in a ratio of one part TGF-b to 1-10,000 parts TNF-a.

Another aspect of the invention is the method of reducing the side effects of TNF or IFN-a treatment in a mammal, which method comprises reducing the dosage of TNF or IFN-a administered, and coadministering a therapeutically effective amount of TGF-b.

The compositions of the invention inhibit the proliferation of cells of the lymphoid and myeloid lineages, and thus are useful for suppressing the immune system. Accordingly, one may use the compositions of the invention for treating cancers of lymphoid and myeloid cells, as well as for treating patients where immune suppression is desired. Disorders associated with cancers of lymphoid and myeloid cells include, but are not limited to, Burkitt's lymphoma, T-cell lymphomas, B-cell lymphomas, and various myelocytic leukemias. Disorders of the immune system include, but are not limited to, autoimmune diseases such as psoriasis, rheumatoid arthritis, osteoarthritis, myasthenia gravis, lupus erythematosus, and uveitis. In addition, the compositions of this invention may be useful in suppressing immune responses against transplanted organs.

The compositions of the invention are also able to inhibit the pathological proliferation of various hyperproliferative cell types, including for example keratinocytes (in the case of mammals suffering from psoriasis).

In the practice of the invention, the compositions of the invention need not be administered simultaneously. It is preferred to administer individual cytokines within 72 hours. Preferably, TNF or IFN-a is administered simultaneously with TGF-b, or TGF-b may be administered within 48 hours of TNF or IFN-a administration.

The compositions of the invention may be used to treat diseases by local application. The route of administration will be dependent upon the disorder being treated. The composition, TGF-b with TNF, may be particularly effective for the local treatment of solid tumor masses. Such composition may be directly injected into superficial tumor masses through a hypodermic needle. Superficial tumor masses include skin cancers such as melanoma or basal cell carcinomas. Deep tumor masses may be treated with TGF-b and TNF through a catheter, e.g., a vascular catheter. This method of administration will be particularly effective for the treatment of tumor masses in the liver, kidney, or brain.

The combination of TGF-b with IFN-a may be applied locally to treat inflammatory disorders. Some examples include topical application of the composition in a suitable carrier system. Topical application may be useful in the treatment of hyperproliferative and inflammatory diseases of the skin, for example atopic dermatitis and psoriasis. Chronic inflammatory diseases of the joints may be treated by injecting these compositions directly into the inflamed joint. This method of administration is useful in the treatment of rheumatoid arthritis and osteoarthritis.

The compositions of the invention may also be used to treat diseases by systemic administration. The combination of TGF-b with TNF may be effective in the systemic treatment of cancer. The dosage of each component required will vary depending on the age and size of the patient, the type of cancer being treated and the severity of the disease. Likewise, the composition, TGF-b with TNF, may be effective for the systemic treatment of autoimmune diseases and for systemic immune suppression.

Although TGF-b, TNF, and IFN-a are all suited for administration by parenteral injection, other forms of administration are also contemplated and are to be considered within the scope of this invention. For example, one may effect administration by inhalation, via intranasal or bronchial spray. Alternatively, the compounds may be delivered directly via numerous controlled-release or sustained-release implant devices. For example, one may incorporate one or more components into a polymer implant or membrane suitable for implantation. Alternatively, one may implant subcutaneously an Alzet® minipump (ALZA Corporation, Palo Alto, CA) to deliver one or more components over an extended period of time.

Most formulations of the invention will be in the form of solutions or suspensions of the active compound(s) in a physiologically acceptable vehicle suitable for parenteral injection, for example, in phosphate-buffered saline (PBS). Other acceptable excipients may also be included, for example dextrose, carboxymethyl cellulose, butylated hydroxyanisole (BHA), butylated hydroxytoluene (BHT), human serum albumin (HSA), polyethyelene glycol (PEG), and the like. For a complete description of suitable formulation techniques and materials, one is referred to "Remington's Pharmaceutical Sciences," by E.W. Martin (Mack Pub. Co., Easton, PA, l5th Ed., 1975).

## EXAMPLES

The following examples are presented as a further guide and illustration of the invention for those of ordinary skill in the art. However, it should be understood that the invention is not limited to the specific embodiments disclosed below, but also includes all equivalents.

## Example 1

### (Preparation of TGF-b)

TGF-b1 and TGF-b2 were purified from demineralized bone powder as in European Patent Application 35304848.6. Briefly, bovine metatarsal bone was demineralized for 16 hr in 0.5 M HCl at 4°C and peptides solubilized utilizing a 4M Gdn-HCl/1 mM N-ethylmaleimide/10 mM EDTA, pH 6.8 extraction procedure. TGF-bs were then purified by gel filtration on Sephacryl® S-200 columns equilibrated in 4 M Gdn-HCl/0.02% sodium azide/10 mM EDTA, pH 6.8 followed by cationic exchange chromatography on CM cellulose using a linear 10-400 mM NaCl gradient in 6 M urea/10 mM NaCl/1 mM N-ethymaleimide/50 mM sodium acetate, pH 4.5. Final purification and resolution of TGF-b1 from TGF-b2 was achieved by reversed phase HPLC on $C_{18}$ columns eluted with 0.60% acetonitrile gradient in 0.1% trifluoroacetic acid, pH 1.9. Homogeneity was demonstrated by silver stained SDS-PAGE analysis and by N-terminal amino acid sequence analysis. The resulting TGF-bs are at least 95% pure and are suitable for use without further purification.

It should be noted that, due to the high degree of homology between TGF-bs obtained from different species, one may substitute TGF-b obtained from other mammals for human TGF-b. For example, one may use fresh porcine or bovine blood as an inexpensive substitute for tissues of human origin.

## Example 2

### (Formulations)

(A) The following is a formulation suitable for administration via intravenous injection:

| TGF-b1 or TGF-b2 | 1 mg to 100 mg |
|---|---|
| IFN-a | 10,000 U to 1,000,000 U |
| HSA | 5.0 mg |
| PBS qs | 1.0 ml |

The components are added to the phosphate-buffered saline, preferably as lyophilized powders, and the pH adjusted to about 7.4. One unit (U) of specific activity is the amount of cytokine (e.g., IFN-a) which induces a half maximal biologic response in vitro.

(B) The following is another formulation suitable for administration via intravenous injection:

| | |
|---|---|
| TGF-b1 or TGF-b2 | 1 mg to 1 g |
| TNF-a | 1 mg to 1 g |
| Dextrose | 2.0 mg |
| Methyl cellulose | 2.5 mg |
| PBS qs | 1.0 ml |

The components are added to the phosphate-buffered saline, preferably as lyophilized powders, and the pH adjusted to about 7.4.

(C) The following is a liposome formulation suitable for administration via intravenous injection:

| Active Components: | |
|---|---|
| TGF-b1 or TGF-b2 | 1 mg to 1 g |
| IFN-a | 10,000 U to 1,000,000U |
| HSA | 5.0 mg |
| PBS qs | 1.0 ml |
| Liposomes: | |
| DAPC | 0.5 ml |
| DAPS | 0.01 ml |
| Cholesterol | 0.02 mg |

The components are added to the phosphate-buffered saline, preferably as lyophilized powders, and the pH adjusted to about 7.4. For liposome formulations, the PBS should be free of Ca++, Mg++, and other divalent cations.The DAPC (diarachadoylphosphatidylcholine), DAPS (diarachadoylphosphatidylserine), and cholesterol are dissolved in 5 ml of chloroform in a round bottom flask. The chloroform is then removed under reduced pressure, leaving a thin lipid film on the sides of the flask. The Active Component solution is then added, and the flask vigorously agitated (e.g., by sonication) to form a suspension of liposomes suitable for intravenous or intramuscular injection. Other suitable methods for preparing liposomal formulations are known in the art.

(D) The following is a formulation suitable for topical administration, e.g., for topical treatment of psoriasis:

| | |
|---|---|
| TGF-b1 or TGF-b2 | 1 mg to 50 mg |
| IFN-a | 100,000 U to 1,000 U |
| SPAN® 60 | 2.0 g |
| TWEEN® 60 | 2.0 g |
| Mineral Oil | 5.0 g |
| Petrolatum | 10.0 g |
| Methyl Paraben | 0.15 g |
| Propyl Paraben | 0.05 g |
| BHA | 0.01 g |
| Water qs | 100 ml |

The components (except for TGF-b, IFN-a, and water) are combined and heated to 60°C with stirring. A quantity of water is then added with vigorous stirring to produce about 90 g of cream, which is then cooled. The TGF-b and IFN-a are then suspended in about 10 ml of water (optionally containing stabilizers such as albumin, dextrose, etc.), and the solution added to the cream mixture. The resulting mixture is then stirred, and stored below room temperature.

Example 3

Bone Marrow Soft Agar Colony Formation Assay

(A) BALB/c mice bone marrow cells were suspended in 1 ml RPMI-1640 (Advanced Biotechnologies, Silver Spring, MD), 10% fetal calf serum (FCS), 0.1 mM 2-mercaptoethanol (2-Me) in 0.3% seaplaque agarose (Rockland, ME) in 35 mm Lus petri dishes (Miles Scientific, Naperville, IL) at 37°C in 5% $CO_2$. Colony formation was induced using murine granulocyte/monocyte colony stimulating factor (GM-CSF). The cultures were treated with 0.1 ng/ml TGF-b with varying amounts of TNF (0.1 to 100 U/ml). The number of colonies were counted at 7 days and the data expressed as the number of bone marrow colonies per plate.

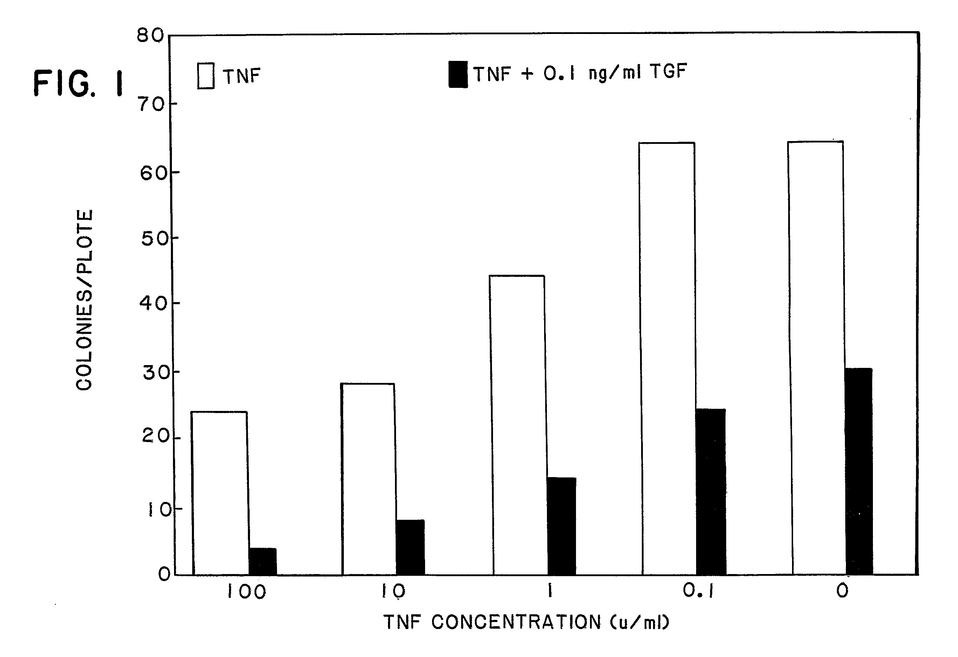

(B) The effect of TGF-b and TNF upon in vitro development of granulocyte-macrophage colony forming units (GM-CFU) from normal murine bone marrow is shown in Figure 1. TNF alone (10-100 U/ml) suppressed GM-CFU development by 35-62%. TGF-b alone suppressed GM-CFU development by 54% at 0.1 ng/ml. The combination of TNF (1-100 U/ml) and TGF-b (0.1 ng/ml) suppressed GM-CFU development by 80-93%. These results show that a combination of TGF-b with TNF effectively suppresses the development of GM-CFU from cultured normal bone marrow.

Example 4

Bone Marrow Cell Proliferation Assay

(A) Bone marrow was aspirated from the femur of BALB/c mice with RPMI-1640, washed twice and resuspended in RPMI-1640 containing 10% FCS and then seeded into 96 well microtiter plates (Costar, Cambridge, MA) at $1 \times 10^5$ cells in 0.1 ml. The cells were induced to proliferate using 5 ng/ml of murine GM-CSF. The experimental cultures were treated with either different amounts of TNF (0.01 to 500 U/ml) alone or TNF (0.01 to 500 U/ml) with 0.1 ng/ml TGF-b. Proliferation was assessed after 72 hr of incubation at 37° C in 5% $CO_2$ with an overnight pulse of 1 mCi of $^3$H-thymidine (New England Nuclear, Boston, MA). Cell cultures were harvested with a multiple sample harvester onto glass fiber filters. Individual filters were placed in 2 ml of PCS scintillation fluid (Amersham Corporation, Arlington Heights, IL), and the samples counted by standard liquid scintillation methods.

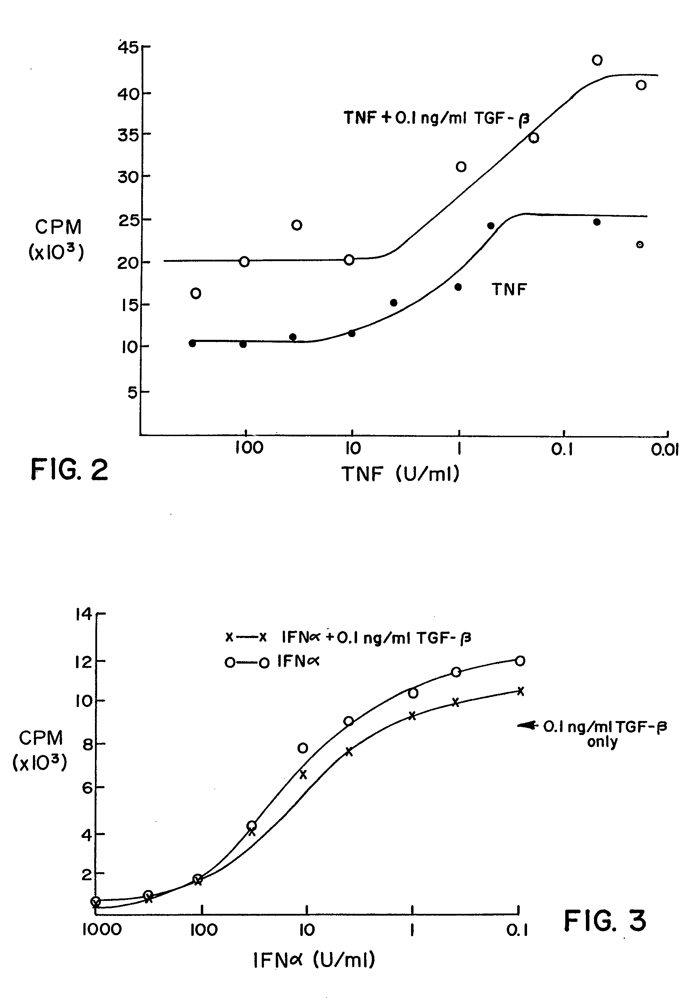

(B) TGF-b and TNF inhibition of GM-CSF-induced growth in the bone marrow proliferation assay is shown in Figure 2. The results show that TGF-b (0.1 ng/ml) greatly enhances the bone marrow suppressive action of TNF. The half maximal effective action dose ($ED_{50}$) of TNF was shifted from 2 U/ml to 0.6 U/ml in the presence of TGF-b (0.1 ng/ml).

(C) The procedure in part A was repeated, substituting IFN-a for TNF. As shown in Figure 3, the combination of TGF-b and IFN-a does improve the suppressive action of IFN-a in the GM-CSF-induced bone marrow proliferation assay, although not to the same degree as with TNF. The result indicates that a combination of TGF-b and TNF is more effective at inhibiting bone marrow progenitor cell growth than a combination of TGF-b and IFN-a.

Example 5

Thymocyte Proliferation Assay

(A) C3H/HeJ murine thymocytes ($10^6$) were plated into flat bottom 96 well microtiter plates (Costar 3596, Cambridge, MA). The cells were costimulated with an optimal concentration of phytohemagglutinin (Sigma Chemical Company, St. Louis, MO) and interleukin-1 (8 U/ml IL-1, Cistron Technology, Pine Brook, NJ). Different amounts of IFN-a (5 to 500 U/ml) with or without different amounts of TGF-b (0.001 to 0.1 pM) were added to the culture. The cells were cultured for 72 hr in a humidified incubator in 5% $CO_2$ at 37° C. Prior to harvesting (24 hr) the cultures were pulsed with 0.5 mCi of $^3$H-thymidine (Amersham, Chicago, IL). These cultures were harvested with a semiautomated cell harvester and the amount of $^3$H-thymidine incorporated was determined by standard liquid scintillation methods. The data are expressed as disintegrations per minute (DPM) from triplicate cultures.

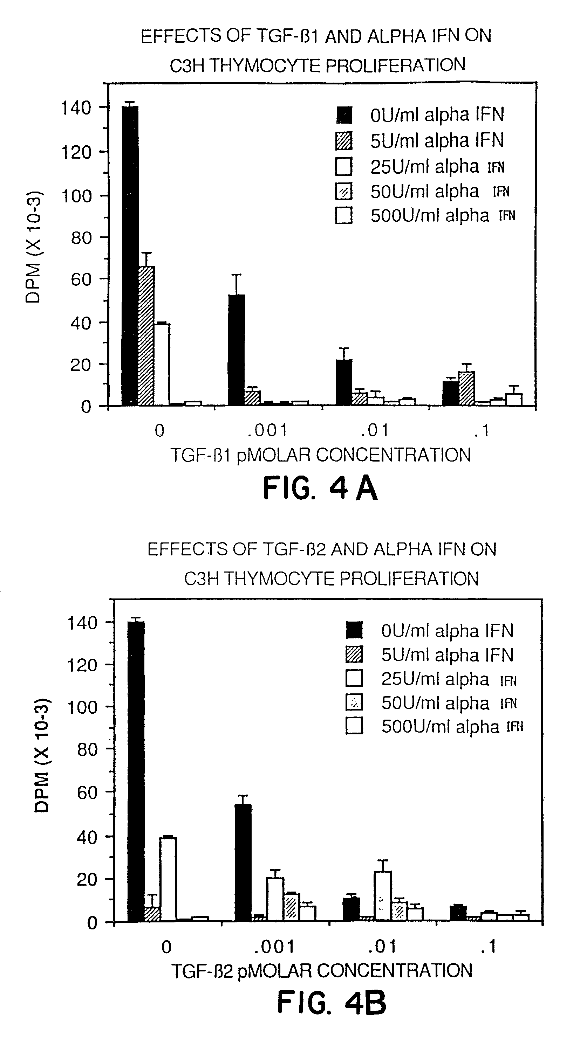

(B) The effect of TGF-b1 combined with IFN-a and the effect of TGF-b2 combined with IFN-a in the IL-1 induced thymocyte proliferation assay are shown in Figures 4a and 4b, respectively. A combination of 0.001 pM TGF-b1 or TGF-b2 with 5 U/ml IFN-a completely (f90%) suppressed T-cell proliferation in this assay.

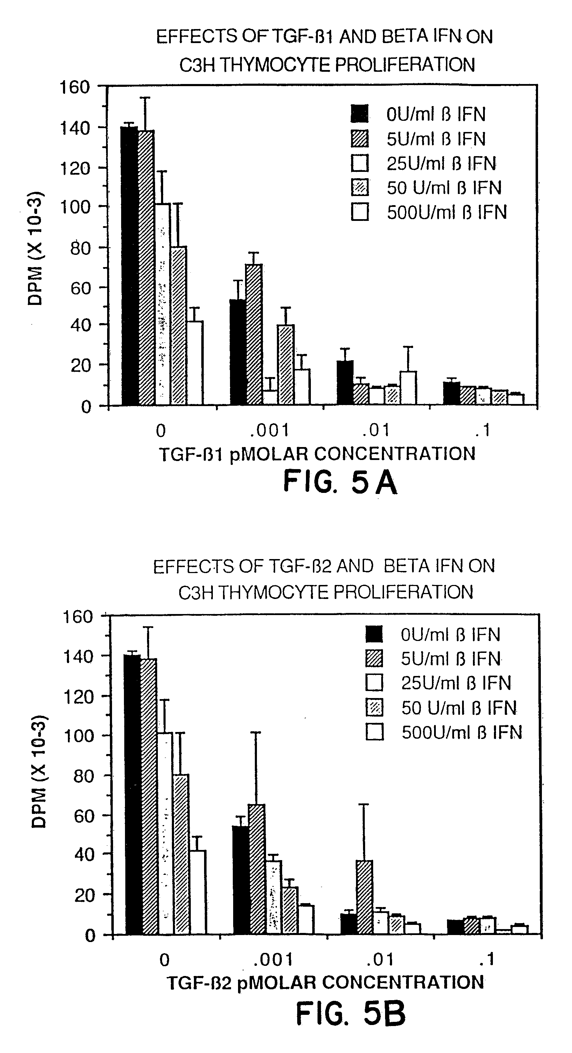

(C) The effects of TGF-b1 or TGF-b2 combined with IFN-b in the IL-1-induced thymocyte proliferation assay are shown in Figures 5a and 5b, respectively. TGF-b1 or TGF-b2 when combined with IFN-b are much less effective at suppressing the proliferative response of T-cells induced with IL-1 in the thymocyte proliferation assay than combinations of TGF-b1 and IFN-a or TGF-b2 and IFN-a.

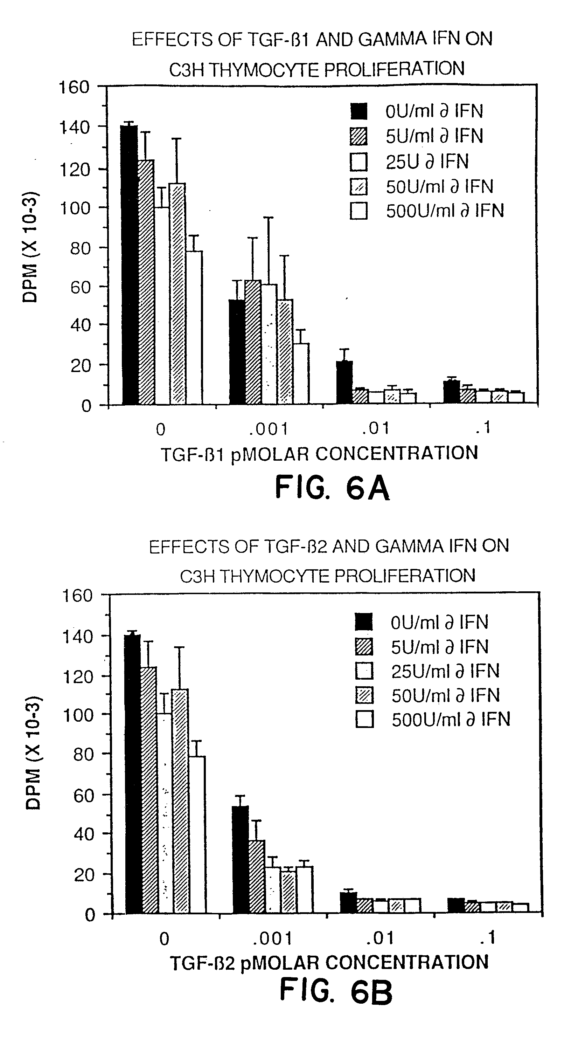

(D) The effects of TGF-b1 combined with IFN-q and TGF-b2 combined with IFN-q are shown in Figures 6a and 6b, respectively. The figures show that combinations of TGF-b1 or TGF-b2 with IFN-q are much less effective at suppressing the proliferative response of T-cells induced with IL-1 in the thymocyte proliferation assay than combinations of TGF-b1 or TGF-b2 with IFN-a.

(E) The results shown in Figures 4, 5, and 6 demonstrate that TGF-b1 or TGF-b2 with IFN-a is the most effective and desired combination of TGF-b and IFN for suppressing T-cell proliferation.

## Example 6

### Leukemic Cell Proliferation Assay

(A) Human histiocytic leukemic cell line, U937, was cultured in Falcon tissue culture flasks in RPMI-1640 supplemented with 10% FCS, penicillin (100 U/ml), streptomycin (100 mg/ml) and 3 mg/ml glutamine. The cells were treated with either TNF alone (0.1 to 1,000 U/ml) or TNF (0.1 to 1,000 U/ml) in combination with 0.1 ng/ml TGF-b. In some experiments, the cells were treated with either IFN-a alone (1 to 1,000 U/ml) or IFN-a (1 to 1,000 U/ml) in combination with 0.1 ng/ml TGF-b. The treated cells were cultured at $10^5$ cells/well in triplicate for a period of 72 hr. Then, the cells were pulsed with 1 mCi $^3$-thymidine for 6 hr prior to harvesting and counting by liquid scintillation methods. The data are expressed as counts per minute (CPM).

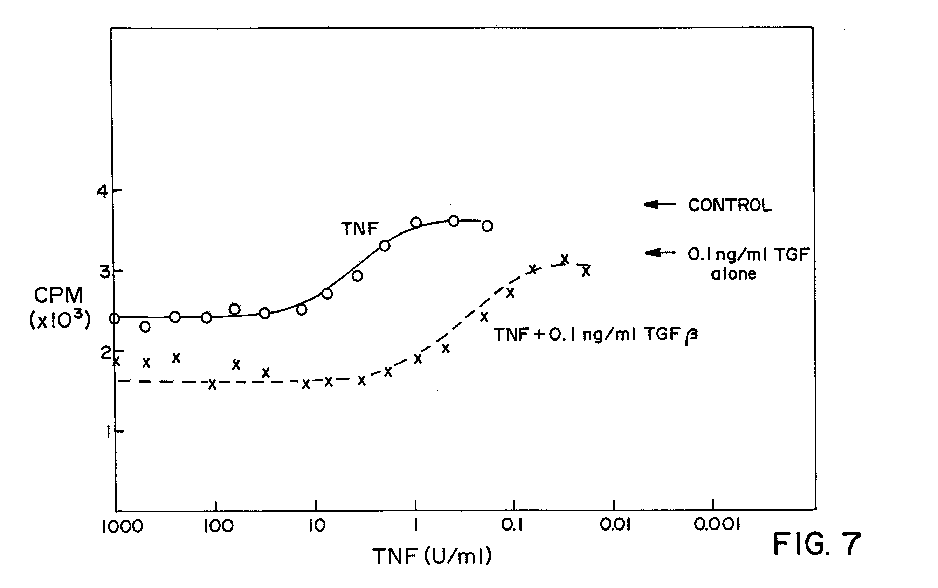

(B) The results of TGF-b with TNF in the leukemic cell proliferation assay is shown in Figure 7. The growth-inhibiting effect of TNF on tumor cells is greatly improved when 0.1 ng/ml TGF-b is added.

## Claims

1. A composition that substantially inhibits the growth of immune cells, hematopoietic progenitor cells, hyperproliferating cells, and cancer cells in a mammal, which composition comprises TGF-b with TNF or with IFN-a.

2. The composition according to claim 1 which is effective for treating hematopoietic progenitor cells.

3. The composition according to claim 1 which is effective for treating immune cells.

4. The composition according to claim 1 comprising an amount of TGF-b and TNF that substantially inhibits the growth of cancer cells.

5. The composition according to claim 4 wherein said cancer cells are malignant cells.

6. The composition according to claim 2, claim 3, claim 4 or claim 5 which comprises a therapeutically effective amount of TGF-b and TNF.

7. The composition according to claim 4 wherein the TNF is TNF-a.

8. The composition according to claim 5 wherein the TGF-b and TNF-a are present in a ratio of 1:1-10,000.

9. The composition according to claim 2 or claim 3 which comprises TGF-b and IFN-a.

10. The composition of claim 9 wherein the TGF-b and IFN-a are present in a ratio of 1:1-10,000.

## Claims for the following Contracting States: ES,GR

1. A method of forming a composition that substantially inhibits the growth of immune cells, hematopoietic progenitor cells, hyperproliferating cells, and cancer cells in a mammal, which method comprises admixing TGF-b with TNF or with IFN-a in the composition.

2. The method according to claim 1 wherein the amounts used are effective for treating hematopoietic progenitor cells.

3. The method according to claim 1 wherein the amounts used are effective for treating immune cells.

4. The method according to claim 1 comprising using an amount of TGF-b and TNF that substantially inhibits the growth of cancer cells.

5. The method according to claim 4 wherein said cancer cells are malignant cells.

6. The method according to claim 2, claim 3, claim 4 or claim 5 which comprises a therapeutically effective amount of TGF-b and TNF.

7. The method according to claim 4 wherein the TNF is TNF-a.

8. The method according to claim 5 wherein the TGF-b and TNF-a are used in a ratio of 1:1-10,000.

9. The method according to claim 2 or claim 3 which comprises using TGF-b and IFN-a.

10. The method of claim 9 wherein the TGF-b and IFN-a are used in a ratio of 1:1-10,000.

11. TGF-b with TNF or with IFN-a, for use in the inhibition of cell growth.

12. Method for the preparation of a pharmaceutical composition for the treatment of cancer or cancer precursors comprising forming said composition with TGF-b and TNF or TGF-b and IFN-a.

FIG. I

□ TNF          ■ TNF + 0.1 ng/ml TGF

COLONIES/PLOTE

80
70
60
50
40
30
20
10
0

100          10          1          0.1          0

TNF CONCENTRATION (u/ml)

EP 0 325 471 A1

FIG. 2

FIG. 3

EFFECTS OF TGF-ß1 AND ALPHA IFN ON
C3H THYMOCYTE PROLIFERATION

FIG. 4 A

EFFECTS OF TGF-ß2 AND ALPHA IFN ON
C3H THYMOCYTE PROLIFERATION

FIG. 4B

EFFECTS OF TGF-ß1 AND BETA IFN ON
C3H THYMOCYTE PROLIFERATION

FIG. 5 A

EFFECTS OF TGF-ß2 AND BETA IFN ON
C3H THYMOCYTE PROLIFERATION

FIG. 5B

EFFECTS OF TGF-ß1 AND GAMMA IFN ON
C3H THYMOCYTE PROLIFERATION

FIG. 6A

EFFECTS OF TGF-ß2 AND GAMMA IFN ON
C3H THYMOCYTE PROLIFERATION

FIG. 6B

FIG. 7

EP 0 325 471 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 224 885 (WAKUNAGA SEIYAKU K.K.) * Page 6, lines 14,20-24; page 65, line 20 - page 67, line 4; claims 1-28 * | 1-12 | A 61 K 37/36 A 61 K 45/02 // (A 61 K 37/36 A 61 K 37:02 ) (A 61 K 45/02 A 61 K 37:36 ) |
| P,X | CHEMICAL ABSTRACTS, vol. 110, no. 11, 13th March 1989, page 533, no. 93274v, Columbus, Ohio, US; R.KAMIJO et al.: "Suppression of TNF-stimulated proliferation of diploid fibroblasts and TNF-induced cytotoxicity against transformed fibroblasts by TGF-beta" & BIOCHEM. BIOPHYS. RES. COMMUN. 1989, 158(1), 155-62 * Abstract * | 1-12 | |
| A | EP-A-0 105 014 (THE UNITED STATES OF AMERICA AS REPRESENTED BY THE SECRETARY UNITED STATES DEPARTMENT OF COMMERCE) & WO-A-84 01 106 (Cat. D,A) | 1-12 | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 19-04-1989 | BRINKMANN C. |